(19) Europäisches Patentamt / European Patent Office / Office européen des brevets

(11) **EP 4 148 737 A1**

(12) **EUROPEAN PATENT APPLICATION**

(43) Date of publication:
**15.03.2023   Bulletin 2023/11**

(21) Application number: **21306260.7**

(22) Date of filing: **14.09.2021**

(51) International Patent Classification (IPC):
**G16B 5/00** (2019.01)    **G16B 40/00** (2019.01)
**C12Q 1/68** (2006.01)    **G01N 33/00** (2006.01)
**G16B 10/00** (2019.01)

(52) Cooperative Patent Classification (CPC):
**G16B 5/00; G16B 10/00; G16B 40/00; G16H 20/60**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Integrative Phenomics**
**75011 Paris (FR)**

(72) Inventors:
- **BELDA CUESTA, Eugenio**
  **78370 Plaisir (FR)**
- **GIORDANO, Nils**
  **44240 La Chapelle sur Erdre (FR)**
- **SWARTZ, Timothy**
  **75012 Paris (FR)**

(74) Representative: **Cabinet Camus Lebkiri**
**25 rue de Maubeuge**
**75009 Paris (FR)**

(54) **COMMUNITY-BASED MICROBIOME MODELLING PROCESS FROM METAGENOMICS DATA AND ASSOCIATED SYSTEM**

(57)    The invention relates to a process for modelling an intestinal microbiome of a patient comprising the steps of:
- receiving a metagenomic dataset resulting from a metagenomic sequencing of a fecal sample of the patient,
- receiving a diet dataset,
- receiving a genome-scale metabolic model for each metagenomic taxon in the metagenomic dataset,
- computing a vector of metabolic fluxes of a community comprising the GSMM linked by at least part of the exchange reactions comprised in the GSMM, the GSMM exchanging molecules with a common luminal compartment of the community, and comprising additional exchange reactions defining release of the molecules by the common luminal compartment in a fecal compartment, the computing comprising using a linear optimization method with maximizing the community biomass as an objective function and the diet dataset as an external constraint, the vector constituting a molecular fingerprint of the metabolic activity of the intestinal microbiome.

FIG. 3

**Description**

**TECHNICAL FIELD**

The technical field of the invention is the field of metagenomics.

The present document concerns a process for modelling a microbiome from metagenomics data and dietary data and an associated system, and in particular a process in which a microbiome is modelled from metagenomics and dietary data so as to obtain a molecular fingerprint of the microbiome to be able to apply machine learning prediction methods to said fingerprint.

**STATE OF THE ART**

In-silico metabolic modelling of microbes permits to translate functional annotations from (meta)genomic sequences into mathematical models of cellular metabolism. From these mathematical models, system behaviors can be studied under different conditions, also called constraints. To do so, two elements are essential. The first element is the genome-scale metabolic model (GSMM) or network (GSMN) of the cellular system that is studied, that is reconstructed from functional annotations of the genes encoded in the genomic sequences. This GSMN is a repository of all biochemical reactions catalysed by cellular enzymes and transport reactions carried out by different cellular transport systems that describes the drain and production of cellular metabolites in the organism under study needed to sustain cellular growth, for example aminoacids needed for protein synthesis, nucleotides needed for DNA replication and transcription, lipids needed to build cellular membranes, cofactors needed for the activity of different enzymes, etc. The second element is the molecular profile of the environment in which the cellular system inhabits, in the form of different compounds that the system has access to in order to grow, for example carbon sources like glucose, maltose, fructose; nitrogen sources like ammonia, aerobic or anaerobic conditions defined by the presence/absence of oxygen, etc. These molecular profiles are encoded in the form of exchange reactions that introduce these compounds in the extracellular environment of the metabolic network at maximum rates defined by the estimated amounts of these compounds in the environment.

With the biochemical and transport reactions of the GSMM and exchange reactions defining environmental inputs, a mathematical model of the cellular metabolism is built by encoding the reactions of the metabolic network in the form of a stoichiometric matrix (S) where rows represent chemical compounds (m compounds), columns represent reactions (n reactions), and indexes represent the stochiometric coefficient of each metabolite in each reaction of the network. Stochiometric coefficients are molar ratios in which substrates are transformed to products within a chemical reaction. The S matrix is the most important structural property of metabolic networks, which describes the architecture and topological properties of the system, and which remains constant across changing conditions that could alter kinetic parameters or reaction rates. With this S matrix, it is possible to define differential equations for each chemical compound of the network. The differential equations describe the net change of metabolite concentrations as a function of time, which is equal to the differences between the sum of all reaction fluxes which produce the chemical compound and the sum of all reaction fluxes which consume it.

$$\frac{dX}{dT} = S * v$$

This system of differential equations incorporates enzyme kinetics through the time derivative, so the vector of reaction fluxes ($v$) will depend on the concentration of metabolites and a number of kinetic parameters that are unknown for many reactions of the network in GSMM. It is in this context where, to overcome this lack of knowledge of kinetic information, the metabolic system is placed in a steady-state condition, which is the most fundamental constraint in the computational modelling of cellular metabolism. This is justified by the fact that metabolic transients in internal chemical compounds are more rapid than both cellular growth rates and dynamic changes in organism environment. As a consequence, metabolic fluxes leading to the formation and degradation of any particular chemical compound must be balanced and their sum should be equal to zero:

$$0 = S * v$$

By assuming this steady state, the above system of differential equations is converted to a set of linear equations from which it is possible to compute the vector of reaction fluxes ($v$) which defines the metabolic state of the system under the defined environmental conditions. However, this system of linear equations is typically undetermined, meaning that there is no unique solution; i.e. no unique vector of reaction fluxes $v$ that satisfies the steady-state condition, but rather a space of feasible flux distributions, i.e. multiple $\nu$ vectors, in the N-dimensional space of reaction fluxes which satisfy the steady-state condition. This space of flux distributions can be more or less wide depending on additional constraints that could be imposed to the behavior of the system in addition to the environmental ones, in the form of maximum and minimum values that different reaction fluxes can achieve derived from experimental data, such as maximum glucose uptake rates computed experimentally with Metabolic Flux Analyses (MFA); zero flux across reactions catalyzed by unexpressed enzymes in transcriptomics or proteomics datasets, etc.

There are different ways to explore the space of feasible flux distributions, among which the most common is through linear optimization of an objective function. Such linear optimization approaches include:

- Flux Balance Analyses, also known as FBA, which tries to find flux distribution across model reactions that maximizes the objective function,

- Parsimonious FBA also known as pFBA, which tries to find flux distribution across model reactions that maximizes the objective function minimizing the overall total flux across model reactions,

- Flux Variability Analyses, also known as FVA, which tries to find the range of feasible flux values across model reactions that maximizes the objective function.

The mentioned mathematical methods (FBA, pFBA, FVA) explore this space of feasible flux distributions by using Linear Programming (LP) mathematic optimization, which allows to find the best flux distribution $\nu$ that optimizes a given objective function, which is defined in the form of a biomass equation. This biomass equation is typically written as an additional reaction in the model that reflect the needs of the cell (aminoacids, nucleotides, lipids, cofactors, eventually macro-molecules such as DNA, RNA, or proteins in certain models) to make one gram of cellular dry weight, and is usually achieved by examining the relevant literature regarding experimental measurements of biomass constituents of the organisms under study or adapting the biomass equation of related organisms if experimental information of the organism under study is scarce. In summary, LP optimization over a metabolic system with maximizing biomass production as an objective function gives the maximal growth yield achievable by the system under a given set of environmental constraints. The environmental constraints are defined by the nutritional inputs as well as the distribution of reaction fluxes ($\nu$) that is behind the cellular growth phenotype, from which it is possible to extract the rates of consumption of nutrients or secretion of cellular metabolites that define the metabolic state of the system.

This approach relies on the strong assumption that cellular systems have optimized their growth performance under a subset of possible environmental conditions during their evolution, so maximization of biomass can be considered a driving principle of metabolic operation. This has been experimentally validated in organisms like *Escherichia coli* or *Helicobacter pylori,* for which it has been shown that experimentally measured growth rates correlate with biomass production rates in model simulations with FBA and cellular biomass as objective function.

This methodology has been mostly applied in the context of systems and synthetic biology to model the physiology of microorganisms under different conditions, gene essentiality prediction, and identification of genetic targets for metabolic engineering. All these applications are limited to single organism modelling.

Analysis of the intestinal microbiome could help understand the individual response to food, as microbiome-processed molecules are absorbed by the host to affect target organs, have local effects on the intestine, are re-transformed and/or metabolized by microbes, and are excreted in stool, so they have a large range of effects on the individual.

There is therefore a need to be able to analyse and model the intestinal microbiome and its different organisms.

## SUMMARY OF THE INVENTION

The present document solves the above-mentioned problems by providing a solution to model the intestinal microbiome of a human patient, the model comprising the different individual species of the intestinal microbiome and their interactions.

According to a first aspect of the invention, this is satisfied by providing a process for modelling an intestinal microbiome of a human patient, the process comprising at least the steps of:

- Receiving at least one metagenomic dataset, the metagenomic dataset resulting from a metagenomic sequencing of a fecal sample of the human patient and comprising a plurality of abundances of metagenomic taxons,

- Receiving at least one diet dataset, the diet dataset comprising at least one abundance of at least one molecule comprised in at least part of a nutritional diet of the patient,

- Receiving at least one genome-scale metabolic model for each metagenomic taxon comprised in the metagenomic dataset, each genome-scale metabolic model comprising exchange reactions defining environmental conditions, an individual biomass of the metagenomic taxon simulating cellular growth, and biochemical reactions and transport reactions encoded by protein coding genes of the genome of the metagenomic taxon,Reconstructing a microbiome community model comprising the genome-scale metabolic models received previously linked by at least part of the exchange reactions comprised in the genome-scale metabolic models, the genome-scale metabolic models exchanging molecules with a common luminal compartment shared by the genome-scale metabolic models of the community, the community model comprising additional exchange reactions defining intake of molecules from the diet to the common luminal compartment and release of the molecules by the common luminal compartment in a fecal compartment,

- Computing a vector of metabolic fluxes of the microbiome community model, the computing of the vector of metabolic fluxes comprising using a linear optimization method with maximizing the community biomass as an objective function and with the diet dataset as an external constraint, the community biomass being the sum of the individual biomasses weighted by the abundances of the metagenomic taxons, the vector of metabolic fluxes comprising a distribution of metabolic fluxes across reactions of the microbiome community model, the vector constituting a molecular fingerprint of the activity of the microbiome under the nutritional diet of the patient.

Thanks to the invention, it is possible to model the intestinal microbiome, including organisms with available GSMN. The modelling can then be used to provide a fingerprint of the chemical profile of the microbiome under different dietary constraints. It is then possible to put these molecular fingerprints in relation with the clinical and phenotypic data of the patient to find predictive biomarkers associated to different conditions combining model simulations with machine learning prediction methods.

Furthermore, by modifying the dietary constraints, i.e. the dietary dataset received, the molecular fingerprint of the microbiome will change, so it is possible to simulate how the microbiome of an individual will react to variations in their dietary profiles, without having to vary the actual diet of the individual. In the context of precision medicine and personalized nutrition, this approach can lead to the prediction of which dietary profiles maximizes the production of beneficial metabolites, such as for example short-chain fatty acids, or minimize the production of detrimental ones, such as for example Trimethylamine, based on the individual microbiome profiles, opening the way to personalized dietary interventions tailored to improve the metabolic state of the individuals by acting over the metabolic activities of the microbiome.

The invention, by providing a fingerprint of the metabolic activity of the intestinal microbiome, also allows to trace specifically the source of chemical compounds from diet to fecal compartment through reaction fluxes in community models. This permits to understand how the intestinal microbiome of the patient works and how to tune the diet of the patient.

The process for modelling an intestinal microbiome of a human patient may also have one or more of the following characteristics, considered individually or according to any technically possible combinations thereof:

- the fingerprint of the metabolic activity of the intestinal microbiome comprises the metabolic fluxes across biochemical reactions of the members of the community and export reactions of chemical molecules consumed from the diet and exported to the fecal compartment by the community.

- the linear optimization method is chosen from Flux Balance Analysis, Parsimonious Flux Balance Analysis, and Flux Variability Analysis.

- the step of construction of a microbiome community model is performed from metagenomic taxon abundances and genome-scale metabolic models collapsed at higher levels of the taxonomic hierarchy than species-level abundances.

- at the step of construction of a microbiome community model, metagenomic gene abundances are derived from non-redundant gene catalogs of the human gut microbiome and genome-scale metabolic models are derived from functional annotations of said non-redundant gene catalogs.

- the construction of a microbiome community model is performed from patient-specific metagenomic taxons obtained from de-novo genome assembly wherein the metagenomic dataset is generated from the fecal sample and the genome-scale metabolic models are obtained from a functional genome annotation.

Another aspect of the invention relates to a computer program product comprising instructions which, when the program is executed by a computer, causes the computer to carry out the process according to the invention.

Another aspect of the invention relates to a computer-readable medium comprising instructions which, when executed by a computer, cause the computer to carry out the method according to the invention.

The invention can have several applications, for example to test the response to different diets by a patient without the patient having to actually apply said diet, or for example to compare several individual's response to the same diet or to compare diets, or any other suitable application.

## BRIEF DESCRIPTION OF THE FIGURES

Other characteristics and advantages of the invention will become clear from the description that is given thereof below, by way of indication and in no way limiting, with reference to the appended figures, among which:

- Figure 1 shows a schematic representation of an embodiment of a process according to the invention.

- Figure 2 shows a system to carry out the process according to the invention.

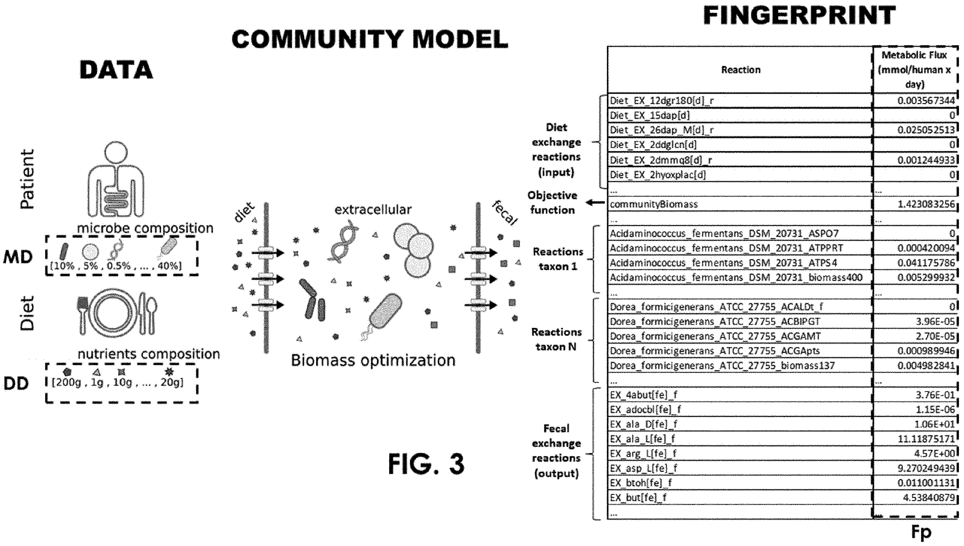

- Figure 3 shows a schematic representation of the invention.

- Figure 4 shows a schematic representation of a community model obtained by a process according to the invention.

## DETAILED DESCRIPTION

For greater clarity, identical or similar elements are marked by identical reference signs in all of the figures.

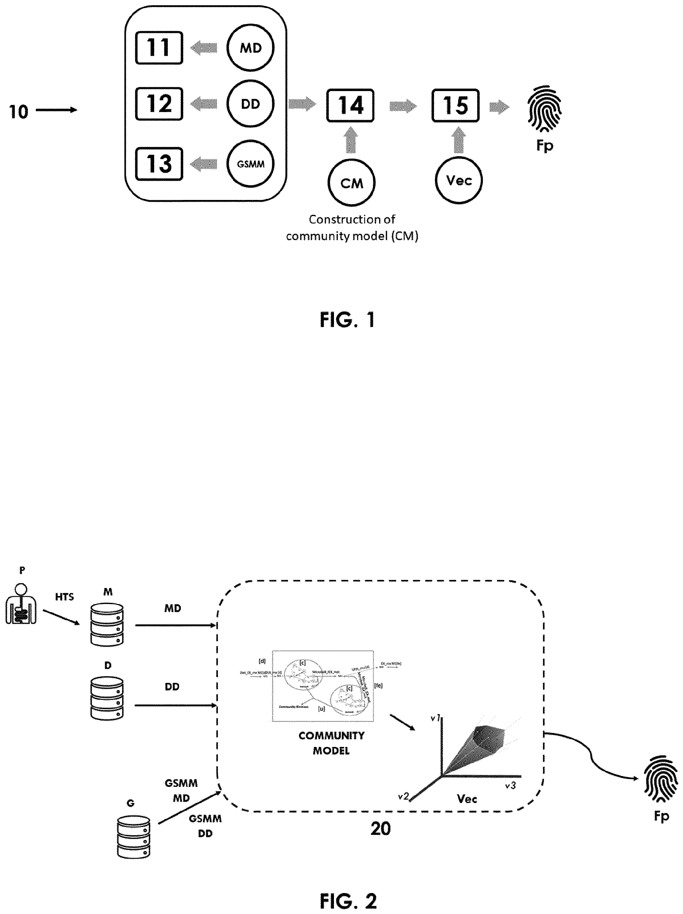

Figure 1 shows a schematic representation of an embodiment of a process according to the invention.

In the first embodiment represented at Figure 1, a process 10 for modelling an intestinal microbiome of a human patient according to the invention comprises five steps. It is understood by "modelling" providing a molecular fingerprint of the activity of the community under a given dietary profile.

The process according to the invention permits to obtain a molecular fingerprint of the intestinal microbiome of a human patient, so as to have an understanding of the functioning of the intestinal microbiome of said human patient and to be able to modify its behavior, for example by modifying the diet in entry.

The process according to the invention comprises a step 11 of receiving at least one metagenomic dataset and a step 12 of receiving at least one diet dataset. The steps 11 and 12 can be performed in any order, for example by receiving the diet dataset before the metagenomic dataset or at the same time. The process 10 represented at Figure 1 is an example of carrying out the invention and the invention can be carried out differently.

The process 10 according to the invention can be carried out by the system 20 represented at Figure 2.

Figure 2 shows a system to carry out the process according to the invention.

The system 20 can be configured to carry out all the steps of the process and/or its different embodiments according to the invention. To do so, the system 20 of Figure 2 comprises a memory and a processing unit, the memory being configured to store instructions which, when executed by the computing unit, cause the computing unit to carry out the steps of the process and/or its different embodiments according to the invention. The memory and the computing of the system 20 are not represented at Figure 2. The system 20 of Figure 2 further comprises at least one network interface to communicate with distant entities, i.e. to send and receive data to said entities, via at least one network. The entities can be servers or computers storing data. The data can be metagenomic data MD, diet data DD, and genome-scale metabolic models GSMM MD and GSMM DD. The data can be stored by the same server or by different servers/databases M, D and G.

At step 11, the metagenomic dataset received results from a metagenomic sequencing of a fecal sample of the human patient and comprises a plurality of abundances of metagenomic taxons. A metagenomic sequencing of a fecal sample comprises a DNA (deoxyribonucleic acid) extraction from a fecal sample, a library construction and a sequencing in order to obtain, after a taxonomic assignment, abundance tables and thus the identification and quantification of the species forming the intestinal microbiome. A metagenomic dataset can be obtained from a fecal sample using a high-throughput sequencing platform such as the NovaSeq® products from Illumina®. A metagenomic dataset is for example one or several abundance tables comprising the abundance of at least one taxon present in the fecal samples. A taxon is understood as being a taxonomic unit, such as for example the bacterium *Akkermansia muciniphila* present in a relative abundance of 1.5% (15000 metagenomic reads classified as A. muciniphila/1000000 total metagenomic reads in the fecal sample). Abundance of taxons can be a relative abundance, for example in percent of microbe composition.

At step 12, a diet dataset is received. The diet data comprises the abundance of at least one molecule comprised in at least part of a nutritional diet of the patient. Abundance of molecules of the diet dataset are absolute nutrients composition in mini-mols of nutrient per human per day. The diet dataset can be obtained from different dietary data collection methods, such as food frequency questionnaires or 24-hours dietary recalls that collect dietary habits of the individuals. Food items in these dietary records are translated into quantitative profiles of macro and micro molecules. The quantitative profile of macro and micro molecules of the diet of the human patient P is what will be called "diet dataset". Preferably, the diet dataset DD comprises the whole quantitative profile. The translation of the food items into a quantitative profile can be done using integrated food databases such as the FoodData Central of the United States Department of Agriculture (https://fdc.nal.usda.gov) which contains the molecular decomposition of these different food items, to be able to define personalized molecular profiles of an individual from their dietary records. The diet dataset will then be used as environmental constraints of the microbiome community model reconstructed from the metagenomic data of the human patient P.

At step 13, genome-scale metabolic models are received for each metagenomic taxon comprised in the metagenomic dataset. Said genome-scale metabolic models can be requested by the system 20 to the database or server G, after receiving the metagenomic dataset at step 11. The system 20 receives in response the genome-scale metabolic models (GSMM), one for each taxon of the metagenomic dataset. The genome-scale metabolic models can be obtained using a repository called AGORA (http://dx.doi.org/10.1038/nbt.3703) which contains GSMM for over 800 microorganisms of the gut microbiome, reconstructions that has been received different degrees of manual curation in terms of reaction content and biomass equation definition that substantially improves its predictive capability in comparison with automatically generated GSMM.

A genome-scale metabolic model of a metagenomic taxon comprises genes, reactions and metabolites. A genome-scale metabolic model permits to simulate, by a computer, cellular growth of the taxon. It defines environmental conditions, exchange reactions and an individual biomass of the metagenomic taxon. It is understood by "exchange reactions" pseudo-reactions that make available different metabolites to the biochemical and transport reactions encoded by protein coding genes of the genome of the taxon. For example an exchange reaction for L-Glucose (EX_Glc_L: =>L_Glc[e]) makes available L-glucose metabolite (L_Glc) in the extracellular environment of the GSMM ([e]), from which specific transport reactions of the GSMM can transport the metabolite to the cytoplasmic/intracellular compartment (for example through ATP-dependent transport system represented by the reaction L_Glc_ABCtrp: L_Glc[e] + ATP[c] => L_Glc[c] + ADP[c] + Pi[c]), and intracellular reactions can transform the metabolite for given metabolic objective (for example in the case of L-glucose the metabolite is channeled through reactions of the central metabolism that allows to generate energy (ATP) and precursors for different biomass constituents). Environmental conditions are nutrients available to simulate cellular growth (for example glucose in the above example) and the individual biomass of the taxon is a specific reaction that reflects the needs of the cell (aminoacids, nucleotides, lipids, cofactors, eventually macromolecules as DNA, RNA, or proteins in certain models) to make one gram of cellular dry weight (cellular growth). As an example, below is shown the biomass equation of the bacterium *A. muciniphila* commonly present in the human gut microbiome:

**0.0030965 nadp[c] + 35.5403 h2o[c] + 0.21909 glu_L[c] + 0.0030965 ACP[c] + 0.0030965 coa[c] + 0.0030965 thmpp[c] + 40.1102 atp[c] + 0.0030965 nad[c] + 0.13541 gtp[c] + 0.0030965 amet[c] + 0.21909 gln_L[c] + 0.016021 dttp[c] + 0.084104 ctp[c] + 0.016021 datp[c] + 0.016021 dctp[c] + 0.016021 dgtp[c] + 0.0030965 adocbl[c] + 0.20083 asp_L[c] + 0.42793 ala_L[c] + 0.50987 gly[c] + 0.0030965 10fthf[c] + 0.0030965 thf[c] + 0.079264 his_L[c] + 0.37539 leu_L[c] + 0.21107 thr_L[c] + 0.076146 cys_L[c] + 0.0030965 mqn8[c] + 0.2467 arg_L[c] + 0.20083 asn_L[c] + 0.1278 met_L[c] + 0.0030965 ca2[c] + 0.010648 pg180[c] + 0.010648 clpn180[c] + 0.010648 pgai17[c] + 0.010648 clpnai17[c] + 0.010648 pgi17[c] + 0.010648 clpni17[c] + 0.0030965 cobalt2[c] + 0.090832 utp[c] + 0.17946 ser_L[c] + 0.0030965 q8[c] + 0.0030965 cu2[c] + 0.28544 lys_L[c] + 0.0030965 fe2[c] + 0.0030965 fe3[c] + 0.0030965 fad[c] + 0.0030965 2dmmq8[c] + 0.0030965 gthrd[c] + 0.15452 phe_L[c] + 0.18435 pro_L[c] + 0.12068 tyr_L[c] + 0.0030965 pheme[c] + 0.025011 colipa[c] + 0.0030965 k[c] + 0.2418 ile_L[c] + 0.025011 udcpdp[c] + 0.0030965 5mthf[c] + 0.0030965 mg2[c] + 0.0030965 mn2[c] + 0.0030965 ptrc[c] + 0.0030965 pydx5p[c] + 0.025011 PGP[c] + 0.010648 pe180[c] + 0.010648 peai17[c] + 0.010648 pei17[c] + 0.0030965 ribflv[c] + 0.0030965 so4[c] + 0.0030965 sheme[c] + 0.0030965 spmd[c] + 0.047202 trp_L[c] + 0.35223 val_L[c] + 0.0030965 zn2[c] + dnarep[c] + proteinsynth[c] + 0.0030965 cl[c] + rnatrans[c] -> 40 h[c] + 39.9969 pi[c] + 0.4846 ppi[c] + 40** adp[c] + 0.0030965 apoACP[c] + 0.0030965 cbi[c] + **biomass[c]** + 0.0030965 dmbzid[c] + 0.025011 PGPm1[c]

At a non-represented step, additional exchange reactions are received, which will be used later to construct a microbiome community model.

The invention then comprises a step 14 of constructing a microbiome community model. The microbiome community model is created by linking the individual GSMM through their exchange reactions. The received individual GSMM exchange molecules between their individual cytoplasmic environment and a luminal compartment shared by all the GSMM of the community. This permits to exchange molecules between GSMM and ultimately to link GSMM. Additional exchange reactions allow the simulation of the release of the molecules in an external compartment such as the fecal compartment. These additional reactions are related to the individual GSMM and can be received during the step 14 or during a previous step, or can be a step in itself (not represented). In another embodiment, this step can be performed from metagenomic taxon abundances and genome-scale metabolic models collapsed at higher levels of the taxonomic hierarchy than species-level abundances, for example genus, family, order, class, phylum. In an embodiment, metagenomic gene abundances are derived from non-redundant gene catalogs of the human gut microbiome and genome-scale metabolic models are derived from functional annotations of said non-redundant gene catalogs. In another embodiment, the construction of a microbiome community model includes patient-specific metagenomic taxons product of de-novo genome assembly with the metagenomic dataset generated from the corresponding fecal sample and the GSMM reconstruction from the functional genome annotation.

At step 15, a vector Vec of metabolic fluxes of the community is computed. It is understood by « community » the set of taxons of the metagenomic data MD. This vector Vec is computed based on the microbiome community model created as described previously. The computing of the vector Vec of metabolic fluxes comprises computing the admissible flux distributions which satisfy the steady state constraint and using a linear optimization method with maximizing the community biomass as an objective function and with the diet dataset as an external constraint. Such linear optimization method can be, as described in the prior art, FBA, pFBA, FVA or any other suitable linear optimization method. The community biomass is the sum of the individual biomasses of the taxons comprised in the received GSMMs of the taxons, weighted by the abundances of the metagenomic taxons. That is, if a metagenomic taxon is present in larger quantity than antoher metagenomic taxon, its individual biomass will participate more to the communicty biomass. The diet dataset is used as an external constraint, that is it defines the metabolites in entry of the community model. The obtained vector Vec of metabolic fluxes comprises a distribution of metabolic fluxes across reactions of the community that maximizes the community biomass, which constitutes the molecular fingerpring of the activity of the community under the defined dietary constraints. Below is shown an example of a vector Vec of metabolic fluxes (column Flux) product of pFBA simulations over a community model of an individual comprising 402479 reactions coming from 252 metagenomic taxons identified in the fecal sample simulated under a dietary profile derived from the individual Food Frequency Questionnaire (FFQ). For simplicity, only 44 reactions are represented corresponding to Diet exchange reactions that define the consumption of dietary metabolites by the community (Diet_EX_ reactions), the communityBiomass reaction corresponding to the objective function that is maximized by the pFBA step, a subset of reactions from the GSMM of two microbial taxons of the community (Acidaminococcus fermetans and Dorea formicigenerans) with their corresponding biomass reaction (biomass400 and biomass137 in Table 1) and a subset of exchange reactions from the lumen to the fecal compartment (EX_ reactions in Table 1).

Table 1 : example of a subset of a vector Vec of metabolic fluxes

| Reaction | Flux |
|---|---|
| Diet_EX_12dgr180[d]_r | 0,00356734 |
| Diet_EX_12dhchol[d] | 0 |
| Diet_EX_12ppd_S[d] | 0 |
| Diet_EX_13ppd[d] | 0 |
| Diet_EX_15dap[d] | 0 |
| Diet_EX_26dap_M[d]_r | 0,02505251 |
| Diet_EX_2ddglcn[d] | 0 |
| Diet_EX_2dmmq8[d]_r | 0,00124493 |
| Diet_EX_2hyoxplac[d] | 0 |
| Diet_EX_2mbut[d] | 0 |
| Diet_EX_2obut[d]_r | 0,04615976 |
| Diet_EX_glc_D[d]_r | 41,2961 |
| ... | |
| communityBiomass | 1,42308326 |
| ... | |
| Acidaminococcus_fermentans_DSM_20731_ASPO7 | 0 |
| Acidaminococcus_fermentans_DSM_20731_ASPT | 0,02862793 |
| Acidaminococcus_fermentans_DSM_20731_ASPt2r_b | 0 |

(continued)

| Reaction | Flux |
|---|---|
| Acidaminococcus_fermentans_DSM_20731_ASPt2r_f | 0 |
| Acidaminococcus_fermentans_DSM_20731_ASPTA_b | 0,03232827 |
| Acidaminococcus_fermentans_DSM_20731_ASPTA_f | 0 |
| Acidaminococcus_fermentans_DSM_20731_ATPPRT | 0,00042009 |
| Acidaminococcus_fermentans_DSM_20731_ATPS4 | 0,04117579 |
| Acidaminococcus_fermentans_DSM_20731_biomass400 | 0,00529993 |
| ... | |
| Dorea_formicigenerans_ATCC_27755_ACALDt_f | 0 |
| Dorea_form icige ne ra ns_ATCC_27755_ACBIPGT | 3,96E-05 |
| Dorea_formicigenerans_ATCC_27755_ACCOAC | 0 |
| Dorea_formicigenerans_ATCC_27755_ACCOACL | 0 |
| Dorea_formicigenerans_ATCC_27755_ACCOALYS2 | 0 |
| Dorea_form icige ne ra ns_ATCC_27755_ACGAMPM_b | 0 |
| Dorea_formicigenerans_ATCC_27755_ACGAMPM_f | 0,00098995 |
| Dorea_formicigenerans_ATCC_27755_ACGAMT | 2,70E-05 |
| Dorea_formicigenerans_ATCC_27755_ACGApts | 0,00098995 |
| Dorea_formicigenerans_ATCC_27755_biomass137 | 0,00498284 |
| ... | |
| EX_4abut[fe]_f | 3,76E-01 |
| EX_4hpro_LT[fe]_f | 0,20818282 |
| EX_5mthf[fe]_f | 0,00032747 |
| EX_ac[fe]_f | 15,9926457 |
| EX_ade[fe]_f | 0,10273857 |
| EX_adocbl[fe]_f | 1,15E-06 |
| EX_ala_D[fe]_f | 1,06E+01 |
| EX_ala_L[fe]_f | 11,1187517 |
| EX_arg_L[fe]_f | 4,57E+00 |
| EX_asp_L[fe]_f | 9,27024944 |
| EX_btoh[fe]_f | 0,01100113 |
| EX_but[fe]_f | 4,53840879 |

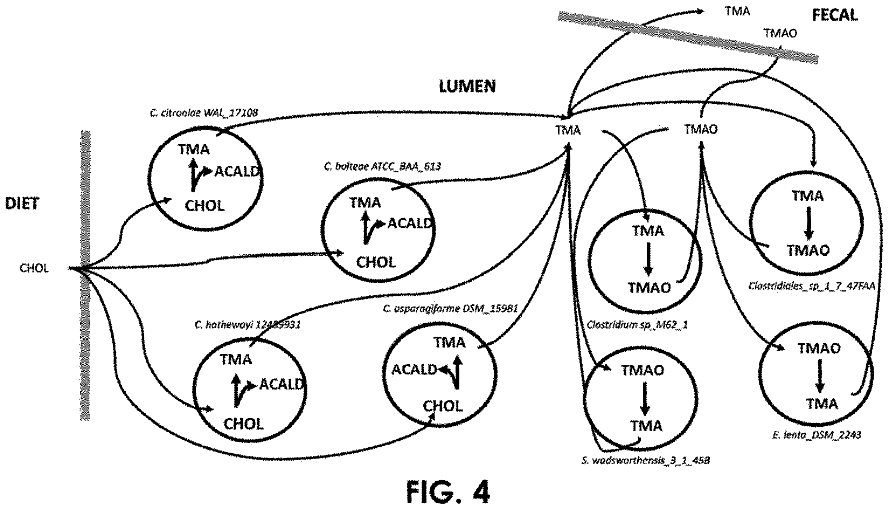

The invention makes it possible to trace specifically the source of chemical compounds from the diet to the fecal compartment through reaction fluxes in the community model, as shown at Figure 4. As represented at Figure 4, it is possible, thanks to the invention, to trace the TMA (trimethylamine) and TMAO (trimethylamine oxide) from the fecal compartment to the dietary source (dietary Choline (CHOL) being the source of TMA/TMAO in the fecal compartment). For example, with a European diet, it can be shown that two bacteria (S. wadsworthensis_3_1_45B and E. lenta_DSM_2243) use luminal TMAO as electron acceptor in respiration, releasing TMA and lowering TMAO in the fecal compartment.

**Claims**

1. Process for modelling an intestinal microbiome of a human patient, the process comprising at least the steps of:

   - Receiving at least one metagenomic dataset, the metagenomic dataset resulting from a metagenomic sequencing of a fecal sample of the human patient and comprising a plurality of abundances of metagenomic taxons,
   - Receiving at least one diet dataset, the diet dataset comprising at least one abundance of at least one molecule comprised in at least part of a nutritional diet of the patient,
   - Receiving at least one genome-scale metabolic model for each metagenomic taxon comprised in the metagenomic dataset and at least one additional exchange reactions, each genome-scale metabolic model comprising exchange reactions defining environmental conditions, an individual biomass of the metagenomic taxon simulating cellular growth, and biochemical reactions and transport reactions encoded by protein coding genes of the genome of the metagenomic taxon,
   - Constructing a microbiome community model comprising the genome-scale metabolic models received previously linked by at least part of the exchange reactions comprised in the genome-scale metabolic models, the genome-scale metabolic models exchanging molecules with a common luminal compartment shared by the genome-scale metabolic models of the community model, the community model comprising the additional exchange reactions defining intake of molecules from the diet to the common luminal compartment and release of the molecules by the common luminal compartment in a fecal compartment,
   - Computing a vector of metabolic fluxes of the microbiome community model, the vector constituting a molecular fingerprint of the activity of the community under the nutritional diet of the patient, the computing of the vector of metabolic fluxes comprising using a linear optimization method with maximizing the community biomass as an objective function and with the diet dataset as an external constraint, the community biomass being the sum of the individual biomasses weighted by the abundances of the metagenomic taxons, the vector of metabolic fluxes comprising a distribution of metabolic fluxes across reactions of the microbiome community model.

2. Process according to the preceding claim **characterized in that** the fingerprint of the metabolic activity of the intestinal microbiome comprises metabolic fluxes across internal biochemical reactions of the community and export reactions of molecules consumed from the diet and exported by the community to the fecal compartment.

3. Process according to any one of the preceding claims **characterized in that** the linear optimization method is chosen from Flux Balance Analysis, Parsimonious Flux Balance Analysis, and Flux Variability Analysis.

4. Process according to any one of the preceding claims **characterized in that** the step of construction of a microbiome community model is performed from metagenomic taxon abundances and genome-scale metabolic models collapsed at higher levels of the taxonomic hierarchy than species-level abundances.

5. Process according to any one of the preceding claims **characterized in that**, at the step of construction of a microbiome community model, metagenomic gene abundances are derived from non-redundant gene catalogs of the human gut microbiome and genome-scale metabolic models are derived from functional annotations of said non-redundant gene catalogs.

6. Process according to any one of the preceding claims **characterized in that** the construction of a microbiome community model is performed from patient-specific metagenomic taxons obtained from *de-novo* genome assembly wherein the metagenomic dataset is generated from the fecal sample and the genome-scale metabolic models are obtained from a functional genome annotation.

7. Computer program product comprising instructions which, when the program is executed by a computer, causes the computer to carry out the process according to any one of the previous claims.

8. Computer-readable medium comprising instructions which, when executed by a computer, cause the computer to carry out the method according to any one of claims 1 to 6.

**FIG. 1**

**FIG. 2**

FIG. 3

**FIG. 4**

Europäisches
Patentamt

European
Patent Office

Office européen
des brevets

## EUROPEAN SEARCH REPORT

Application Number

**EP 21 30 6260**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Baldini Federico: "DEVELOPING INDIVIDUAL-BASED GUT MICROBIOME METABOLIC MODELS FOR THE INVESTIGATION OF PARKINSON'S DISEASE-ASSOCIATED INTESTINAL MICROBIAL COMMUNITIES", , 12 December 2019 (2019-12-12), XP055893064, University of Luxembourg, ?Belvaux, ??Lussemburgo Retrieved from the Internet: URL:https://orbilu.uni.lu/bitstream/10993/41182/1/PhD%20Thesis%20Federico%20Baldini.pdf [retrieved on 2022-02-17] * p. 12, Fig. 1.1; p. 31, Fig. 2.1 * ----- | 1-8 | INV. G16B5/00 G16B40/00 C12Q1/68 G01N33/00 G16B10/00 |
| | | | TECHNICAL FIELDS SEARCHED (IPC) |
| | | | G16B C12Q G01N |

The present search report has been drawn up for all claims

| Place of search | Date of completion of the search | Examiner |
|---|---|---|
| Munich | 18 February 2022 | Heidrich, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................

& : member of the same patent family, corresponding document

EPO FORM 1503 03.82 (P04C01)